# EUROPEAN PATENT APPLICATION

(11) **EP 3 246 064 A1**
(43) Date of publication of application: **22.11.2017**
(21) Application number: 16183423.9
(22) Date of filing: 09.08.2016
(51) Int. Cl.: A61M 16/06

(54) **NASAL CANNULA**

(30) Priority: 16.05.2016 US 201662336900 P
(71) Applicant: L'AIR LIQUIDE, SOCIETE ANONYME POUR L'ETUDE ET L'EXPLOITATION DES PROCEDES GEORGES CLAUDE, 75007 Paris (FR)
(72) Inventor: Bolanger, Thierry, PA 19081 (US)
(74) Representative: Pittis, Olivier

(57) **Abstract**

The invention concerns a nasal gas-delivery system (21) to be connected to a nose of a patient for delivering a respiratory gas to the patient's nostrils, comprising a hollow body (22) with an inner chamber (22a) and an inlet (22b) for receiving a respiratory gas, and a pair of hollow nasal prongs (23, 24), each nasal prongs comprising at least one inner channel (23b, 23c ; 24b, 24c), in fluid communication with the inner chamber 22a of the hollow body (22). The pair of nasal prongs (23, 24) is surrounded by a pair of flexible flange structures (25, 26), the flange structures (25, 26) being joined by a flexible separation wall (27). The flexible flange structures (25, 26) and the flexible separation wall (27) are configured and sized so as to come into contact with outer portions of the nose of the patient thereby creating a gas tight connection in-between, when the nasal gas-delivery is connected to the nose of the patient with the pair of nasal prongs (23, 24) inserted into the patient's nostrils. The nasal gas-delivery system is suitable for delivering gas in the frame of a nasal high flow therapy.

## Description

The invention concerns a nasal gas-delivery system comprising two prongs that are inserted into the patient's nostrils for delivering a respiratory gas to a patient in need thereof, and sealing collars or flanges which allows improving the gas sealing the nasal gas-delivery system and the nose of a patient, especially useable in the frame of a nasal High Flow therapy.

Nasal High Flow Therapy or NHFT is a widespread therapy used in many hospitals, especially in acute care services, against respiratory distresses. It does not require any mechanical ventilation. It can prevent patients from being intubated.

Basically, NHFT consists in providing a humidified, high flow of respiratory gas to a patient through a nasal cannula. Gaseous flows typically range from 10 to 60 L/min for adults and 1 to 10 L/min for infants.

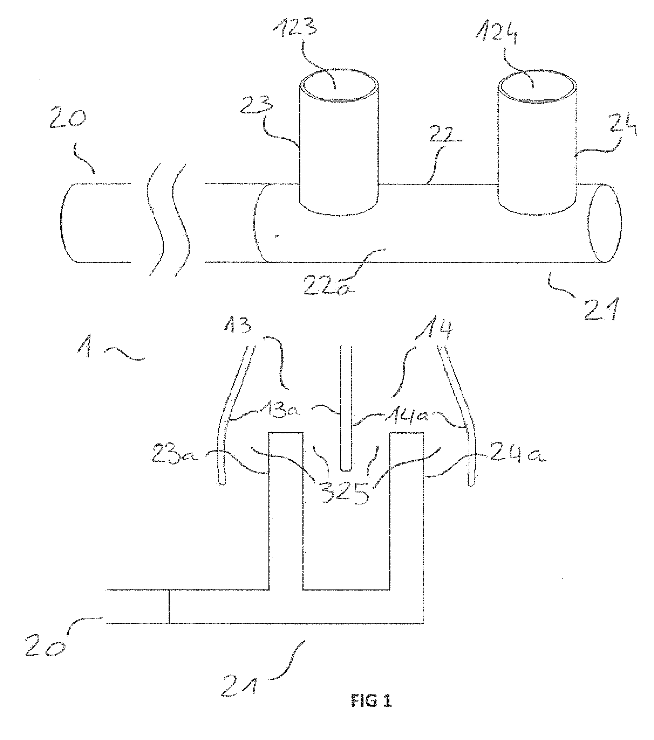

The nasal cannula comprises two prongs that are inserted into the patient's nostrils, although not totally occluding them as shown in Figure 1. The prongs deliver the gaseous flow that the patient inhales and the space between the outer portion of the prongs and the nostrils helps the expired gaseous flow to be vented upon exhalation.

NHFT provides many advantages compared to continuous oxygen treatments or even to non-invasive ventilation, e.g.:
- the gaseous flow can be enriched with Oxygen (O₂) up to 100%, i.e. the respiratory gas can be composed of air, O₂-enriched air (>22 vol% of O₂) or pure O₂.
- the gaseous flow can in some instances be composed of a mixture of O₂ and helium, especially in the pediatric population, i.e. in infants suffering from acute bronchiolitis.
- the set flow can be greater than the inspiratory peak flow of the patient so as to limit his work of breathing, thereby assisting preventing fatigue of respiratory muscles.
- the flow helps flushing out CO₂ from the dead space of the upper airways, thereby enhancing the ventilation.
- the flow and the space between the prongs and the nostrils create a positive pressure, which exhibits a beneficial action on ventilation.

Further, NHFT is extremely simple and leads to other advantages such as:
- only a flow and a level of oxygen have to be set by the physician or other medical service provider;
- the nasal cannula is easy to be put in place; and
- the patient can speak and even eat, while receiving the therapy.

However, some limitations still exist with NHFT because of the non-sealed interface. Indeed, the spaces that exist between the nasal prongs and the nostrils of the patient lead to some important drawbacks, such as:
- the expiratory gases are "lost" and therefore the physician can lose valuable data such as the expiratory volume and by deduction the respiratory rate.
- those gas losses prevent the deployment of Helium/Oxygen-based NHFT as recovery and recycling/reinhalation of helium during a subsequent inhalation phase is not possible. This negatively affects the cost of the therapy as helium is an expensive gas.

US 8,333,200 discloses a nasal cannula which can be used in NHFT, the nasal cannula having an inhalation port outside of the nose and nasal prongs inserted in the nose used as the expiratory port. The expiratory port is connected to a pressure regulator. Such cannula has a primary purpose to limit the pressure down to safe levels and is not intended to create an effective seal. Each nasal prong, indeed, is made of a tubular section, as seen in the prior art and different nose geometries will yield to improper sealing. One further disadvantage of such cannula is that the inhalation port is outside the nose. The flushing effect of NHFT, when the gas is directly injected in the nostrils, will be lost as most of the gas will take the easiest pathway, e.g. down to the pressure regulator. This can lead to significantly different clinical outcomes.

It is a goal of the present invention to provide an improved nasal gas-delivery system, namely a sealed interface, for delivering a respiratory gas or gas mixture to the nostrils of a patient and allowing the recovery of exhaled gases by the patient, especially in the course of a NHFT.

One solution according to the present invention concerns a nasal gas-delivery system or device to be connected to a nose of a patient for delivering a respiratory gas to the patient's nostrils, comprising:
- a hollow body with an inner chamber and an inlet for receiving a respiratory gas, namely a gas or a mixture of several gases, and
- a pair of hollow nasal prongs, each nasal prongs comprising at least one inner channel in fluid communication with the inner chamber of the hollow body, and
- wherein the pair of nasal prongs is surrounded by a pair of flexible flange structures,
- wherein the flange structures are joined by a flexible separation wall, and
- wherein the flexible flange structures and the flexible separation wall are configured and sized so as to come into contact with outer portions of the nose of the patient thereby creating a gas tight connection in-between, when the nasal gas-delivery is connected to the nose of the patient with the pair of nasal prongs inserted into the patient's nostrils.

Depending on the embodiment, a nasal gas-delivery system according to the present invention can comprise one or more of the following features:
- the flange structures have a collar-like structure.
- the flexible flange structures are configured and sized so as to come into contact with the wings, also called 'ala', of the nose of the patient.
- the separation wall is configured and sized so as to come into contact with the columella of the nose of the patient.
- each nasal prong comprises a pair of inner channels, each inner channel comprising a first channel and a second channel arranged in parallel.
- each first channel fluidly connects the internal chamber of the hollow body with a nostril of the patient, and each second channel fluidly connecting a nostril with a vent conduit arranged in the hollow body and in fluid communication with the atmosphere via at least one venting port.
- the vent conduit of the hollow body is in fluid communication with the internal chamber of the hollow body via at least one of the inner channels.
- the prongs have a tronconical general shape
- the flexible flange structures are made of silicone.

Other features, aspects and advantages of the present invention will become apparent from the following detailed description when taken in conjunction with the accompanying drawings which illustrates, by way of examples, the present invention, among which:
- Fig. 1 represents a nasal interface for High Flow Therapy according to the prior art,
- Fig. 2 represents an embodiment of a nasal gas-delivery system according to the present invention, and
- Fig. 2a shows side views (left and right) of the nasal gas-delivery system of Fig. 2,
- Fig. 2b shows a top view of the nasal gas-delivery system of Fig. 2,
- Fig. 3 shows the inner design of the nasal gas-delivery system of Fig.2,
- Fig. 3a, 3b and 3c show the sealing elements of the nasal gas-delivery system of Fig. 2 for different nose profiles of patients,
- Fig. 4 shows a further embodiment of the nasal gas-delivery system of the present invention including an inflatable collar for enhancing the sealing properties of the nasal gas-delivery system of Fig. 2,
- Fig. 4a and 4b show the inner design of the nasal gas-delivery system of Fig.4,
- Fig. 5 shows an embodiment of the nasal gas-delivery system of the present invention that includes a reservoir to collect nasal secretions, and
- Fig. 5a represents an additional view of the inner design of the nasal gas-delivery system of Fig.5

The nasal cannula of Figure 1 according to the prior art comprises an elongated flexible tubing **20,** such as a flexible hose, comprising, at one end, a nasal sub-system **21** to be connected to the nose **1** of a patient for delivering a respiratory gas to the patient. The elongated flexible tubing **20** is connected, by its other ends, to a high flow generator that provides the respiratory gas.

The nasal sub-system **21** comprises a hollow body **22** with an inner chamber **22a,** acting as a manifold for receiving the respiratory gas conveyed and fed by the flexible tubing **20,** and nasal prongs **23, 24,** i.e. little nozzles, in fluid communication with the lumen of the inner chamber **22a** of the hollow body **22,** that are inserted, in use, into the nostrils of the patient for delivering a respiratory gas to the patient in need thereof.

Each nasal prong **23, 24** comprises a unique inner channel or passage **123, 124** for conveying the gas from the inner chamber **22a** of the hollow body **22** to the nostrils **13, 14** of the nose **1** of the patient.

The hollow body **22** can have various shapes, for example be of circular or rectangular cross section. The gas fed by the tubing **20** travels successively in the chamber **22a** of the hollow body **22** and then in each nasal prongs **23, 24,** and eventually is distributed into the nostrils **13, 14** of the patient.

With such a prior art equipment, a efficient gas tight connection/insertion of the prongs **23, 24** into the nostrils **13, 14** cannot be ensured as the outer peripheral walls **23a, 24a** of the nasal prongs **23, 24,** respectively, do not cooperate with, i.e. not perfectly match, the inner walls **13, 14** of the nostrils **13, 14,** i.e., in a manner that provides a sealed contact in-between. This means that a spacing **325** always exists between the inner walls **13a, 14a** of the nostrils **13, 14,** and the outer peripheral walls **23a, 24a** of the nasal prongs **23, 24,** respectively, which spacing **325** leads to gas loses.

Indeed, the gaseous flow in the tubing **20** enters into the inner chamber **22a** of the hollow body **22** and is then directed to the prongs **23, 24** so that upon inhalation, only a part of the gaseous flow is inspired by the patient **1,** as the rest is unfortunately vented to the atmosphere, while escaping through the spacing **325** that inevitably exists.

Upon expiration, both gaseous flows coming, on the one hand, from the high flow generator and, on the other hand, exhaled by the patient do circulate in said spacing **325,** thereby creating a positive expiratory pressure before being vented to the atmosphere.

Such gas loses are not acceptable.

Further, such architecture is not ideal either as, due to the poor air tightness and the gas loses, it is not possible to precisely measure the expiratory volume (and similarly the inspiratory volume) and/or the respiratory rate of the patient. This is a huge drawback as these parameters are very important in respiratory care therapies as they can give some guidance to the physician. Recovering such parameters would only be possible if the interface was a sealed one.

Furthermore, such prior art nasal systems prevents the use of highly effective but costly therapies, for instance He/O₂ mixtures. Thus, mixtures of Helium/Oxygen have proven to be very efficient for treating some respiratory diseases, for instance for alleviating dyspnea in infants having asthma flares or bronchiolitis. As Helium does not cross the lung membrane and is evacuated in the expired gases, it is mandatory for minimizing the overall costs of the therapy to be able to recover the expired gases for recycling He contained therein, which is not possible if the interface is not a sealed one.

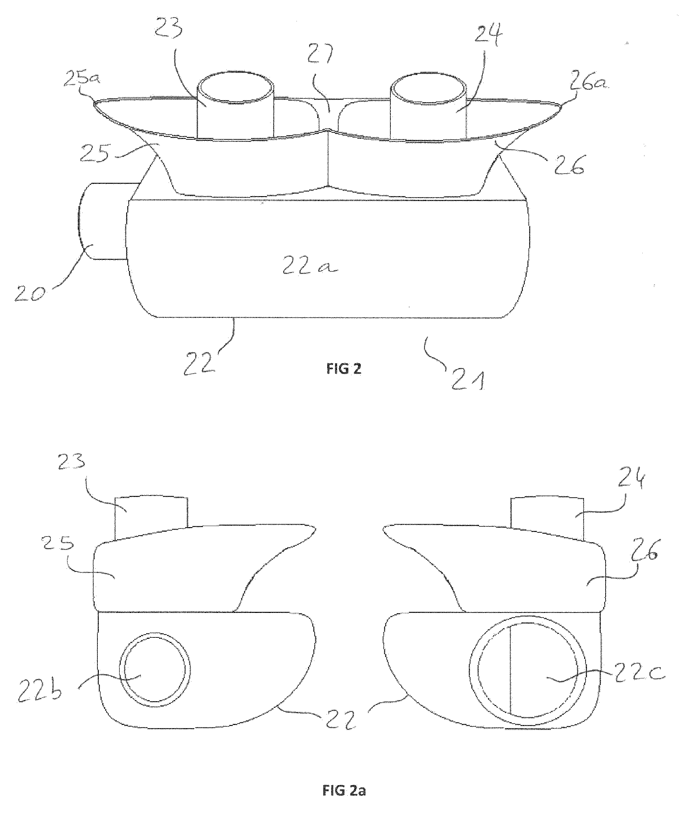

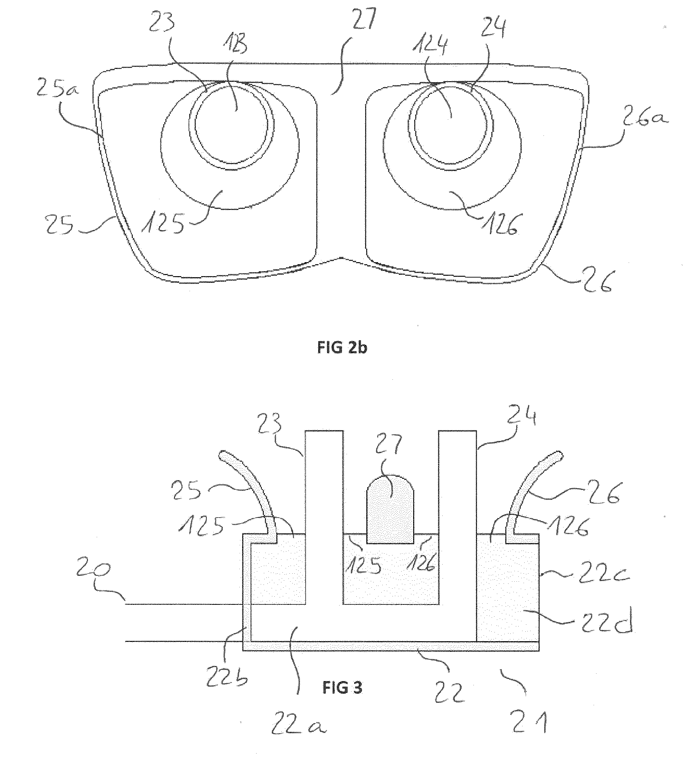

Figures 2, 2a and 2b show a first embodiment of a nasal gas-delivery system **21,** also called nasal sub-system, according to the present invention. It comprises a hollow body **22** having any suitable shape, for example a circular or rectangular section, a gas inlet **22b** and a gas outlet **22c.** The inlet **22b** receives a respiratory gas fed by the flexible tubing **20,** whereas the outlet **22c** recovers the expiratory gas expired by the patient.

The nasal gas-delivery system **21** further comprises two nasal prongs **23, 24** which deliver the gas to the patient's nostrils through the conduits **123, 124.**

According to the present invention, the nasal gas-delivery system **21** further comprises two deformable flanges **25, 26** separated by a common wall **27.** Those elements cooperate with the external surface of the nose so as to obtain a tight seal and further allowing recovering the expired gas in conduits **125, 126.**

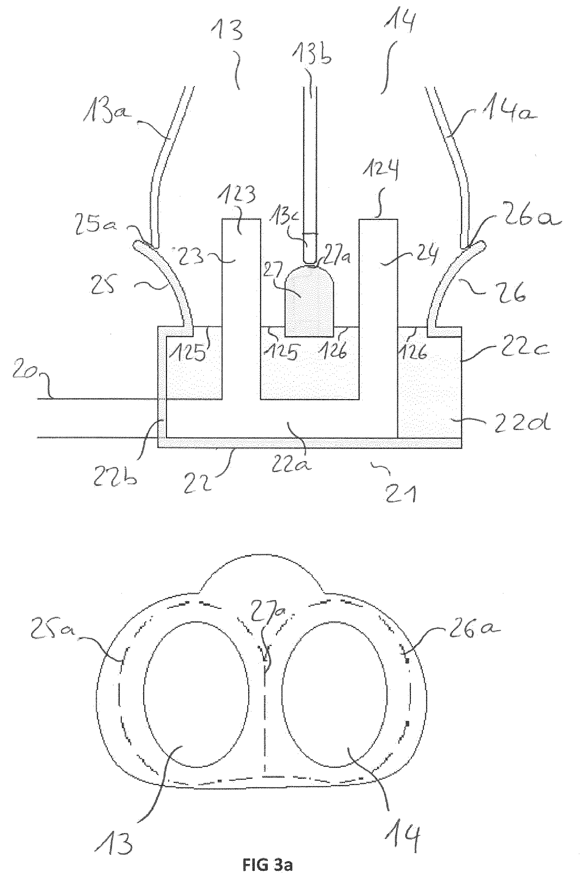

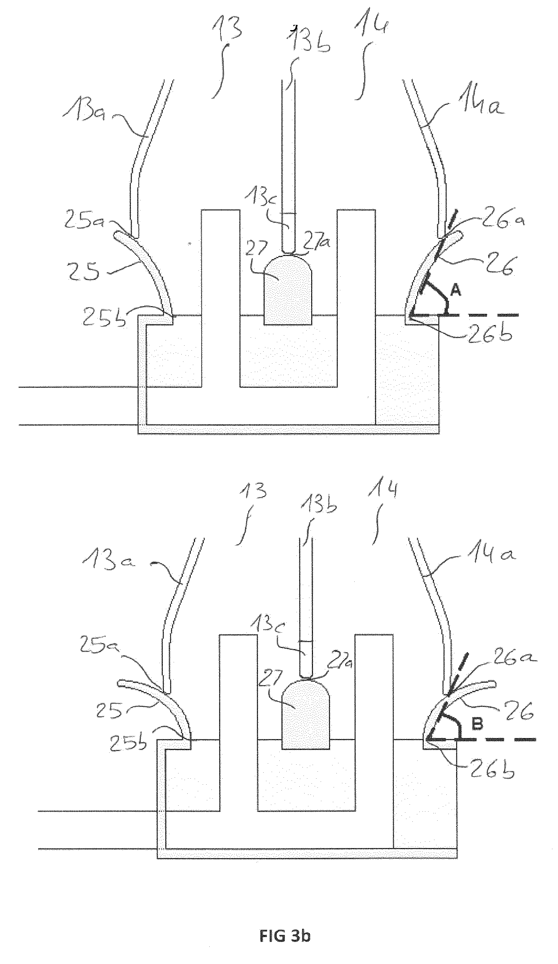

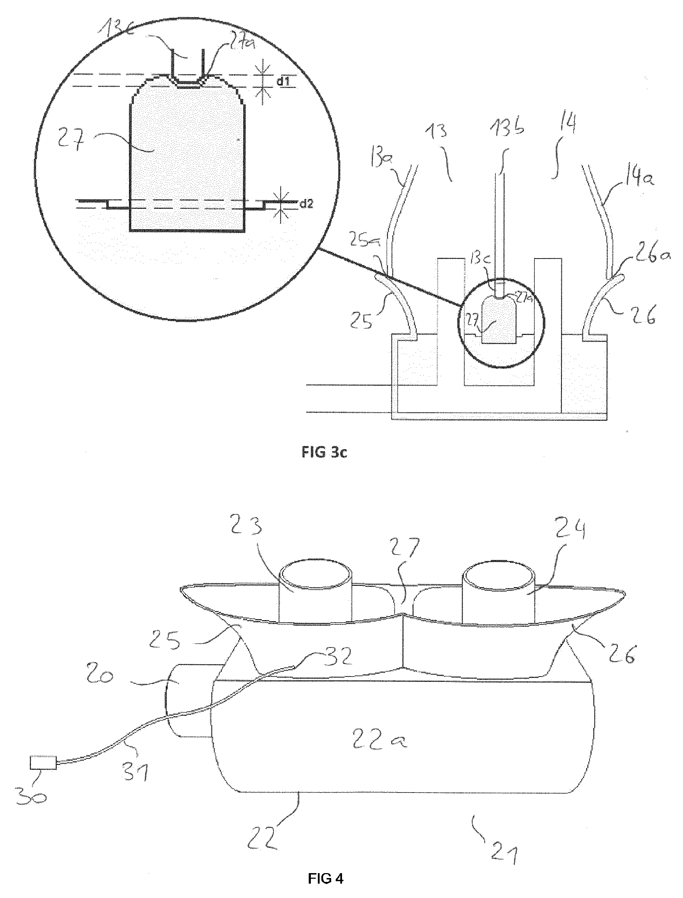

Figure 3 is a cross-sectional schematic view of the nasal gas-delivery system **21** of Figure 2, showing the fluidic connections and internal gas passages. The inlet **22b,** which receives the gas from the flexible tubing **20,** is in fluid communication with the inner chamber **22a** which in turn, is in fluid communication with the inner channels of the two prongs **23, 24.** Similarly, the conduits **125, 126** are in fluid communication with a second inner chamber **22d** which directs the gas to the outlet **22c.** From the outlet **22c,** it is possible to collect the gases provided that the flanges **25, 26** perform a tight sealing with the patient's nose.

Figure 3a represents (cross section view) the nasal sub-system **21** of Figure 2 inserted into the patient's nostrils **13, 14.** The nostrils comprise outer walls **13a, 14a** also called "ala" or "wing" of the nose, and the nasal septum **13b** ending by the columella **13c.**

The first deformable/flexible flange **25** comes into contact (in **25a**) with the ala **13a,** whereas the second deformable/flexible flange **26** comes into contact (in **26a)** with the other ala **14a** of the nose of the patient.

Further, the deformable/flexible wall **27** comes into contact (in **27a**) with the columella **13c** of the nose.

The contact areas **25a, 26a, 27a** between the first and second deformable/flexible flanges **25, 26,** the deformable/flexible wall **27** and the different regions of the patient's nose, lead to a gas sealing around the orifices of the nostrils **13, 14.**

With such a configuration, the gaseous flow, such as air, conveyed by the tubing **20** enters into the internal chamber **22a** of the hollow body **22,** travels through the conduits **123** and **124** of the pair of prongs **23, 24** and is then delivered to the nostrils **13, 14** of the patient.

During the inhalation phases of the patient, a part of the gaseous flow is inspired by the patient, whereas the rest of the gaseous flow is directed to the conduits **125, 126** which are fluidly connected to the vent conduit **22d** thereby allowing this excess of gas escaping to the atmosphere by venting port(s) **22c.**

In contrast, during the exhalation phases of the patient, both gaseous flows coming from the internal chamber **22a** of the hollow body **22,** on the one hand, and exhaled by the patient, on the other hand, pass through the conduits **125, 126** and are vented to the atmosphere thanks to the vent conduit **22d** and venting port **22c,** thereby creating a positive expiratory pressure or PEP.

The nasal sub-system **21** can be made of soft silicon material so as to be light for the patient. Further, it preferably comprises one or several straps, a headgear or similar a fixing system (not shown) for maintaining the nasal sub-system **21** in position in the nostrils **13, 14** of the patient.

Figure 3b which is similar to Figure 3a, shows how the deformable or flexible nature of the flanges **25, 26** can accommodate different nose profiles or morphologies.

As shown, the flanges **25, 26** and the wall **27** create a seal around the inlet orifices of the nostrils **13, 14,** i.e. the external peripheral borders of the nostrils including the wings or alas. The flanges **25, 26** have a determined bending, represented by angle A resulting from the intersection of a line passing by the stem of flange **26b,** the point of contact with the ala **14a** in **26a,** and a horizontal line passing by **26b.**

In case the nose of the patient as longer alas **13a, 14a** (but a 'same' or 'regular' columella **13c**), the flanges **25, 26** will have to bend further so as create a seal around the nostrils **13, 14.** This has been represented by the bending and angle B (determined in the same way as angle A) that is smaller than angle A.

Figure 3c shows how the deformable nature of the wall **27** that can accommodate different nose profiles. In this case, the nasal septum **13b** and columella **13c** are longer than in the above case. The wall **27,** which is made of soft silicon, is flexible and hence capable of deforming so as to fit/match to the size of the columella **13c** for creating a gas seal **27a,** i.e. good gas tightness. This is obtained by a deformation of the upper part of the wall **27** with a maximum displacement **d1** compared to its steady state and further a depression of the bottom part of the wall **27** with a maximum displacement **d2** compared to its steady state.

A nasal gas-delivery system according to the present invention can accommodate different nose profiles and/or morphologies including those having differences of both nasal septum **13b,** columella **13c** and alas **13a, 14a.**

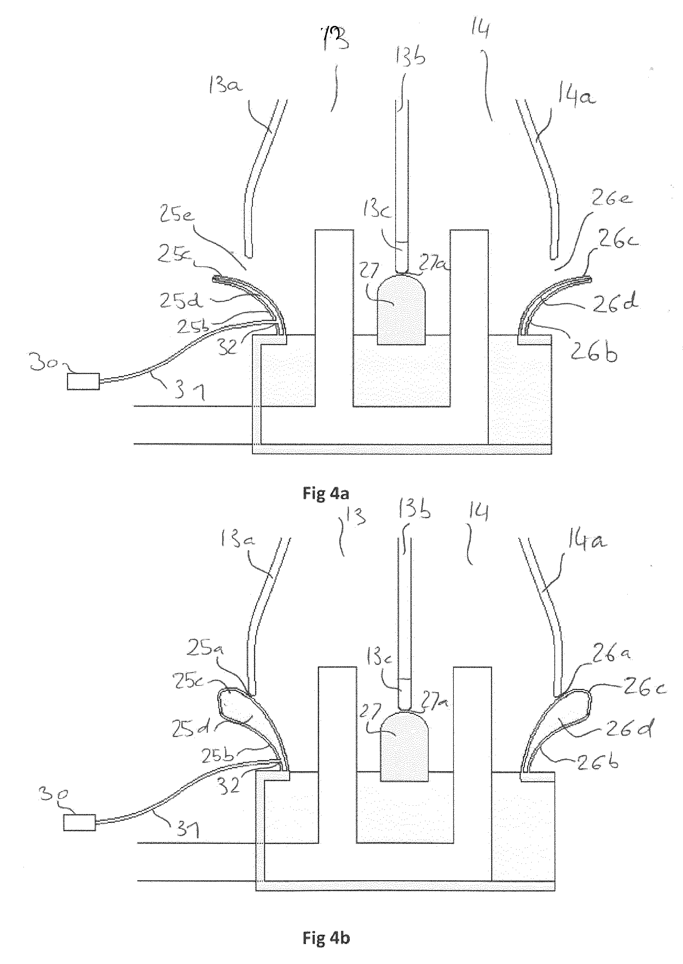

Figures 4, 4a and 4b show another embodiment of the present invention, which is similar to the one of Figure 2 except that it comprises an additional line made of the parts **30, 31, 32** in fluid communication with the flanges **25, 26.**

Figure 4a is a cross-sectional view of Figure 2. The port **30** is an access port to a syringe or any other pressurizing device, for example, port **30** can be similar to the access port used to inflate the cuff of an endotracheal tube. Port **30** is connected to a flexible tubing **31** which ends with a tight connection **32** to the flange **25.** Flange **25** is a hollow part made of two outer walls **25b, 25c** and an inner chamber **25d.** The tight connection **32** has a fluidic relation with the inner chamber **25d.** The first outer wall **25b** is made of a semi-rigid silicone structure, whereas outer wall **25c** is made of a very thin and soft material that allows great amplitude of deformations. Flange **26** has exactly the same structure as flange **25,** e.g. two outer walls **26b, 26c** and an inner chamber **26d** which is in fluid communication with inner chamber **25d** (not shown). Hence, any action having an effect on flange **25** and inner chamber **25d** will have a similar or same effect on flange **26** and inner chamber **26d.**

As shown in Figure 4a, the columella **13c** is in tight connection **27a** with wall **27,** whereas spacings **25e, 26e** exist between alas **13a, 14a** and the upper outer walls **25c, 26c.**

Figure 4b shows an inflated state of the flanges **25, 26.** Upon pressurization through port **30,** for example by a syringe or any other pressurizing device, the flexible tubing **31** and tight connection **32** connect the port **30** to the inner chamber **25d.** As the flange **25** is made of an outer wall **25b** made of a semi-rigid silicone and an outer wall **25c** made of a softer and thinner material, the inflation of the inner chamber **25d** will create an upward motion until the outer wall **25c** comes in tight connection **25a** with the ala **13a.** As inner chambers **25d, 26d** are in fluid communication, the same will occur in flange **26** with an upward motion of the outer wall **26c** until it comes in tight connection **26a** with ala **14a.** A minimum pressure of between 10 and 30 cm H₂O is sufficient to create enough displacement to efficiently seal different nose profiles. Wall **27** behaves in a similar way for allowing its inflation and enhancing the sealing properties of the nasal gas-delivery system **21** of the present invention.

Thanks to a nasal gas-delivery system **21** according to the present invention, it is possible to improve High Flow Therapies in creating an efficient seal around the patient's nostrils thereby improving the gas exchanges between the nasal gas-delivery system and the airways of the patients.

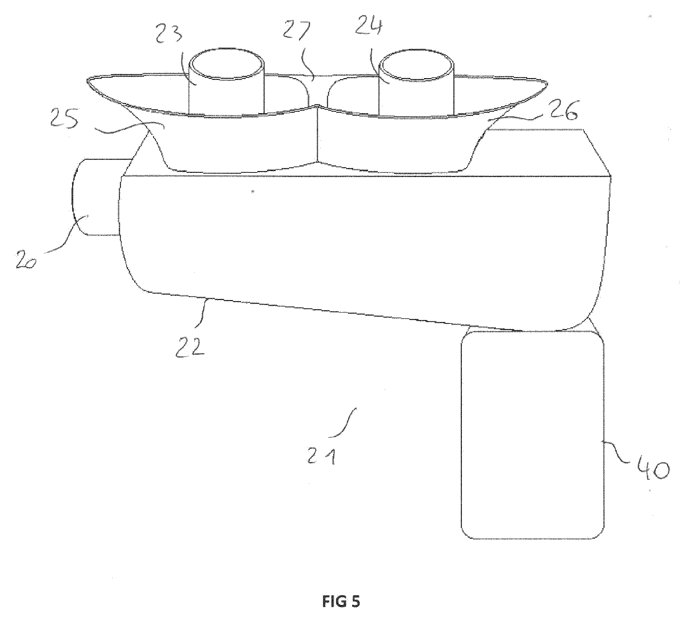

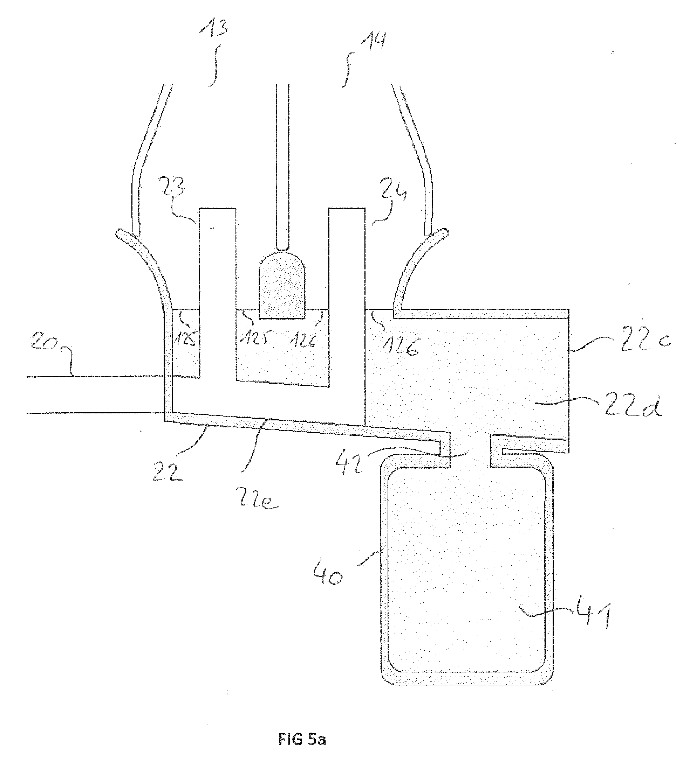

Figure 5 is another embodiment of the present invention, in which the nasal gas-delivery system **21** according to the present invention further comprises a trap **40** to collect the mucus in excess and avoid the patient to have to stop his/her therapy for cleaning the device. Indeed, in HF therapy, the gas has to be highly humidified for avoiding nasal dryness and discomfort for the patients. As a consequence, mucus secretions can be abundant, which constitutes an important drawback as the patient to frequently stop his/her therapy for cleaning his/her nose. The presence of a trap **40** for easily collecting the mucus is highly desirable.

Figure 5a is a cross sectional view of the nasal gas-delivery system of Figure 5 showing the internal design of the mucus collection elements.

As above explained, during the exhalation phases, the gas delivered by the flexible tubing **20** to the cannula **23, 24** and to the port **22c,** are vented through the conduits **125, 126.** Exhalation gases from the patient follow the same ways, whereas mucus can penetrate into the conduits **125** and **126** of the nasal cannula.

To prevent the clogging of the inner chamber **22d** of the hollow body **22** by mucus coming from the nostrils **13, 14** of the patient, the hollow body **22** of the nasal gas-delivery system of the present invention is designed/configured so as to present a slope or ramp **22e** oriented so as to direct the mucus or other fluids toward a mucus evacuation conduit **42,** in which the mucus can enter and then fall/flows by gravity into a collecting reservoir or trap **41,** where it can be recovered and removed.

## Claims

1. A nasal gas-delivery system (21) to be connected to a nose of a patient for delivering a respiratory gas to the patient's nostrils, comprising:
- a hollow body (22) with an inner chamber (22a) and an inlet (22b) for receiving a respiratory gas, and
- a pair of hollow nasal prongs (23, 24), each nasal prongs comprising at least one inner channel (123; 124), in fluid communication with the inner chamber 22a of the hollow body 22, and
- wherein the pair of nasal prongs (23, 24) is surrounded by a pair of flexible flange structures (25, 26),
- wherein the flange structures (25, 26) are joined by a flexible separation wall (27), and
- wherein the flexible flange structures (25, 26) and the flexible separation wall (27) are configured and sized so as to come into contact with outer portions of the nose of the patient thereby creating a gas tight connection in-between, when the nasal gas-delivery is connected to the nose of the patient with the pair of nasal prongs (23, 24) inserted into the patient's nostrils.

2. The nasal gas-delivery system according to Claim 1, wherein the flexible flange structures (25, 26) are configured and sized so as to come into contact with the wings (13a, 14a) of the nose of the patient.

3. The nasal gas-delivery system according to Claim 1, wherein the separation wall (27) is configured and sized so as to come into contact with the columella (13c) of the nose of the patient.

4. The nasal gas-delivery system according to Claim 1, wherein each nasal prong (23, 24) comprises a pair of inner channels (123, 125; 124, 126), each inner channels (123, 125; 124, 126) comprising a first channel (123; 124) and a second channel (125; 126) arranged in parallel.

5. The nasal gas-delivery system according to Claim 4, wherein :
- each first channel (123; 124) fluidly connects the internal chamber (22a) of the hollow body (22) with a nostril (13, 14) of the patient (1), and
- each second channel (125; 126) fluidly connecting a nostril (13, 14) with a vent conduit (22d) arranged in the hollow body (22) and in fluid communication with the atmosphere via at least one venting port (22c).

6. The nasal gas-delivery system according to Claim 1, wherein the vent conduit (22d) of the hollow body (22) is in fluid communication with the internal chamber (22a) of the hollow body (22) via at least one of the inner channels (123, 125 ; 122, 124).

7. The nasal gas-delivery system according to Claim 1, wherein the prongs (23, 24) have a conical or tronconical general shape.

8. The nasal gas-delivery system according to Claim 1, wherein the flexible flange structures (25, 26) are made of silicone.

9. The nasal gas-delivery system according to Claim 1, wherein it further comprises a fixing system for maintaining the nasal prongs (23, 24) in position into the patient's nostrils (13, 14).

## Amended claims

### Amended claims in accordance with Rule 137(2) EPC.

1. A nasal gas-delivery system (21) to be connected to a nose of a patient for delivering a respiratory gas to the patient's nostrils, comprising:
- a hollow body (22) with an inner chamber (22a) and an inlet (22b) for receiving a respiratory gas, and
- a pair of hollow nasal prongs (23, 24), each nasal prongs comprising at least one inner channel (123; 124), in fluid communication with the inner chamber (22a) of the hollow body (22), and
- wherein the pair of nasal prongs (23, 24) is surrounded by a pair of flexible flange structures (25, 26),
- wherein the flange structures (25, 26) are joined by a flexible separation wall (27), and
- wherein the flexible flange structures (25, 26) and the flexible separation wall (27) are configured and sized so as to come into contact with outer portions of the nose of the patient thereby creating a gas tight connection in-between, when the nasal gas-delivery is connected to the nose of the patient with the pair of nasal prongs (23, 24) inserted into the patient's nostrils
**characterized in that**:
- the flexible flange structures (25, 26) are configured and sized so as to come into contact with the wings (13a, 14a) of the nose of the patient, and
- the separation wall (27) is configured and sized so as to come into contact with the columella (13c) of the nose of the patient.

2. The nasal gas-delivery system according to Claim 1, wherein each nasal prong (23, 24) comprises a pair of inner channels (123, 125; 124, 126), each inner channels (123, 125; 124, 126) comprising a first channel (123; 124) and a second channel (125; 126) arranged in parallel.

3. The nasal gas-delivery system according to Claim 2, wherein :
- each first channel (123; 124) fluidly connects the internal chamber (22a) of the hollow body (22) with a nostril (13, 14) of the patient (1), and
- each second channel (125; 126) fluidly connecting a nostril (13, 14) with a vent conduit (22d) arranged in the hollow body (22) and in fluid communication with the atmosphere via at least one venting port (22c).

4. The nasal gas-delivery system according to Claim 1, wherein the vent conduit (22d) of the hollow body (22) is in fluid communication with the internal chamber (22a) of the hollow body (22) via at least one of the inner channels (123, 125 ; 122, 124).

5. The nasal gas-delivery system according to Claim 1, wherein the prongs (23, 24) have a conical or tronconical general shape.

6. The nasal gas-delivery system according to Claim 1, wherein the flexible flange structures (25, 26) are made of silicone.

7. The nasal gas-delivery system according to Claim 1, wherein it further comprises a fixing system for maintaining the nasal prongs (23, 24) in position into the patient's nostrils (13, 14).
